# EUROPEAN PATENT SPECIFICATION

(11) **EP 1 983 929 B1**
(45) Date of publication and mention of the grant of the patent: **23.11.2011**
(21) Application number: 06717153.8
(22) Date of filing: 13.02.2006
(51) Int. Cl.: A61F 2/06, A61L 27/00, B05D 5/08, C08G 18/00, C08L 75/00

(54) **ENDOVASCULAR DEVICE WITH MEMBRANE**
ENDOVASKULÄRE VORRICHTUNG MIT MEMBRAN
DISPOSITIF ENDOVASCULAIRE AVEC MEMBRANE

(43) Date of publication of application: 29.10.2008
(73) Proprietor: Merlin MD PTE Ltd, Singapore 757716 (SG)
(72) Inventor: RUDAKOV, Leon, Singapore 757716 (SG); HONG, Tsui Ying, Rachel, Singapore 757716 (SG); SUNG, Peir Fen, Singapore 757716 (SG)
(74) Representative: Hill, Justin John
(86) International application number: PCT/SG2006/000028
(87) International publication number: WO 2007/094738

(56) References cited:
- EP-A1- 0 864 301
- EP-A2- 0 815 806
- EP-A2- 1 121 911
- EP-A2- 1 543 798
- WO-A1-00/48517
- WO-A1-01/87184
- WO-A1-2005/094725
- WO-A1-2005/094726
- WO-A2-02/22024
- US-A- 4 100 309
- US-A- 5 026 607
- US-A- 5 632 772
- US-A- 6 017 577
- US-A1- 2002 045 931
- US-A1- 2005 171 593
- US-B1- 6 265 016

## Description

### Technical Field

The invention concerns an endovascular device for insertion into a bodily vessel to treat a diseased, damaged or weakened portion of a vessel wall.

### Cross-reference to related applications

### Title: A Medical Device.

### Application No.: PCT/SG2004/000407. Filed: 13 December 2004.

### Publication No.: WO 2005/094726 A1. Published: 13 October 2005.

### Inventors: Leon Rudakov, Michael O'Connor and Deepak Gandhi.

### Background of the invention

The reaction of a bodily vessel when a foreign body is implanted in the bodily vessel is generally negative. Implantation of the foreign body causes cells to react defensively leading to an inflammatory response, and neointimal proliferation which results in narrowing and occlusion of the bodily vessel.

Polyurethanes have been used in long term implants, but have not resolved the abovementioned problem in endovascular treatments, especially in small blood vessels. Small blood vessels are considered to be those with an inner diameter of 2.0 to 4.5mm.

A permeable and porous membrane for a medical device for insertion into a bodily vessel to treat an intracranial aneurysm is disclosed in the previously filed cross- related application, the contents of which are herein incorporated by reference.

EP 0815806 A2 discloses a covered stent for treating a stenotic region in a blood vessel. The stent covering has different porosities in different regions. In those regions, typically the ends, where tissue ingrowth and re-endothelialization are desired, the stent covering is more porous, and in those regions were it is desirable to inhibit such ingrowth, the stent covering is substantially non-porous.

### Summary of the Invention

In a preferred aspect, there is an endovascular device for insertion into a bodily vessel to treat a diseased, damaged or weakened portion of a vessel wall, the endovascular device comprising: a mechanically expandable device expandable from a first position to a second position, said mechanically expandable device is expanded radially outwardly to the second position such that the circumferential surface of said mechanically expandable device engages with the inner surface of the vessel so as to maintain a fluid pathway through said vessel; and a membrane covering at least a portion of the circumferential surface of said mechanically expandable device; wherein the membrane has equidistant porosity and pore sizes from about 1 to 40 µm, bridge dimensions from about 10 to 100 µm, and a material ratio, or proportion of material coverage with respect to total surface area of the membrane, from 75% to 100% and wherein the membrane is made from a biocompatible highly elastomeric polymer, the polymer being a polyurethane based material with end groups.

There may be provided an endovascular device for insertion into a bodily vessel to treat a diseased, damaged or weakened portion of a vessel wall, the endovascular device comprising:
a mechanically expandable device expandable from a first position to a second position, said mechanically expandable device is expanded radially outwardly to the second position such that the circumferential surface of said mechanically expandable device engages with the inner surface of the vessel so as to maintain a fluid pathway through said vessel; and
a membrane covering at least a portion of the circumferential surface of said mechanically expandable device, the membrane comprising a plurality of pores, the porosity of the membrane being defined by the ratio of the material surface area of the membrane determined by the size of the pores and the distance between adjacent pores;
wherein the mechanically expandable device is positioned in the bodily vessel such that the membrane covers at least the diseased, damaged or weakened portion of the vessel wall, the porosity of the membrane obstructing blood supply to the diseased, damaged or weakened portion of the vessel wall and enhancing healing of the bodily vessel.

The membrane may be made from a biocompatible highly elastomeric polymer.

The polymer may be polyether urethane (PEU) or polycarbonate urethane (PCU).

The polymer may be a polyurethane based material with an end group, the end group being any one from the group consisting of: fluorocarbon surface-modified end groups, and polyethylenglycol and silicon surface-modified end groups.

The membrane is a micro-porous membrane.

The porosity of the macro-porous membrane may permit blood supply to perforators of main arteries.

The porosity of the micro-porous membrane may enable enhanced endothelial cell migration and tissue in-growth for faster endothelialization.

The size of each pore may be from about 1 to 30 [mu]m for the micro-porous membrane. The distance between adjacent pores is from about 10 to 100 [mu]m for the micro-porous membrane. The ratio of the material surface area of the membrane is from about 75% to 100% for a micro-porous membrane.

The pores are spaced equidistant from each other.

The pores may be spaced apart at a first distance to each other at a first region and further spaced apart at a second distance to each other at a second region. The size of each pore at a first region may be smaller than the size of each pore at a second region. The first region may be adjacent to the diseased, damaged or weakened portion of the vessel wall.

The first region may encounter blood flow before the second region based on the direction of blood flow in the bodily vessel.

There may be provided an endovascular device for insertion into a bodily vessel to treat a diseased, damaged or weakened portion of a vessel wall, the endovascular device comprising: a mechanically expandable device expandable from a first position to a second position, said mechanically expandable device is expanded radially outwardly to the second position such that the circumferential surface of said mechanically expandable device engages with the inner surface of the vessel so as to maintain a fluid pathway through said vessel; and a membrane covering at least a portion of the circumferential surface of said mechanically expandable device; wherein the mechanically expandable device is positioned in the bodily vessel such that the membrane covers at least the diseased, damaged or weakened portion of the vessel wall; and wherein the membrane is made from a biocompatible highly elastomeric polymer, the polymer being a polyurethane based material with end groups to minimise narrowing of the bodily vessel after the endovascular device is inserted into the bodily vessel.

The end group may be any one from the group consisting of: fluorocarbon surface-modified end groups, and polyethylenglycol and silicon surface-modified end groups. A lubricious layer may be applied to the exterior surface of the membrane and/or exterior circumferential surface of the mechanically expandable device to reduce friction between the membrane and/or mechanically expandable device with the vessel wall of the bodily vessel.

The lubricious layer may be made from a polymer from any one from the group consisting of: hydrophilic polyvinylpyrrolidone, polyacrylate, polymethacrylate, hydrogels, polyethylene oxide and gelatin.

The material of the membrane may be modified using the polymer of the lubricious layer such that predetermined surface properties of the membrane are obtained.

Radiopaque markers may be positioned on the mechanically expandable device to enable alignment of the membrane to the diseased, damaged or weakened portion of the vessel wall.

The diseased, damaged or weakened portion of the vessel wall may be any one from the group consisting of: intracranial aneurysm, saccular aneurysm, wide neck aneurysm, fusiform aneurysm, caroticocavemous fistula, and arteriovenous malformation (AVM).

The bodily vessel may have an inner diameter of about 2.0 to 4.5mm.

There may be provided a porous membrane for an endovascular device to be inserted into a bodily vessel for treating a diseased, damaged or weakened portion of a vessel wall, the membrane comprising: a plurality of pores, the porosity of the membrane being defined by the ratio of the material surface area of the membrane determined by the size of the pores and the distance between adjacent pores; wherein the membrane covers at least the diseased, damaged or weakened portion of the vessel wall, and the porosity of the membrane obstructs blood supply to the diseased, damaged or weakened portion of the vessel wall and enhances healing of the bodily vessel.

### Brief Description of the Drawings

An example of the invention will now be described with reference to the accompanying drawings, in which:
Figure 1 is a graphical illustration of the characteristics of pores of the membrane of an endovascular device in accordance with a preferred embodiment of the present invention;
Figure 2 is a graphical illustration of equally spaced apart pores;
Figure 3 is a graphical illustration of a macro-porous membrane for an endovascular device in accordance with a preferred embodiment of the present invention;
Figure 4 is a graphical illustration of a micro-porous membrane for an endovascular device in accordance with a preferred embodiment of the present invention;
Figure 5 is a side view of the endovascular device inserted into a bodily vessel and the blood flow from the bodily vessel into the aneurysm;
Figure 6 is a top plan view of the endovascular device;
Figure 7 is an image an aneurysm located in the subclavian artery of a rabbit;
Figure 8 is an image of the endovascular device against the aneurysm shown in

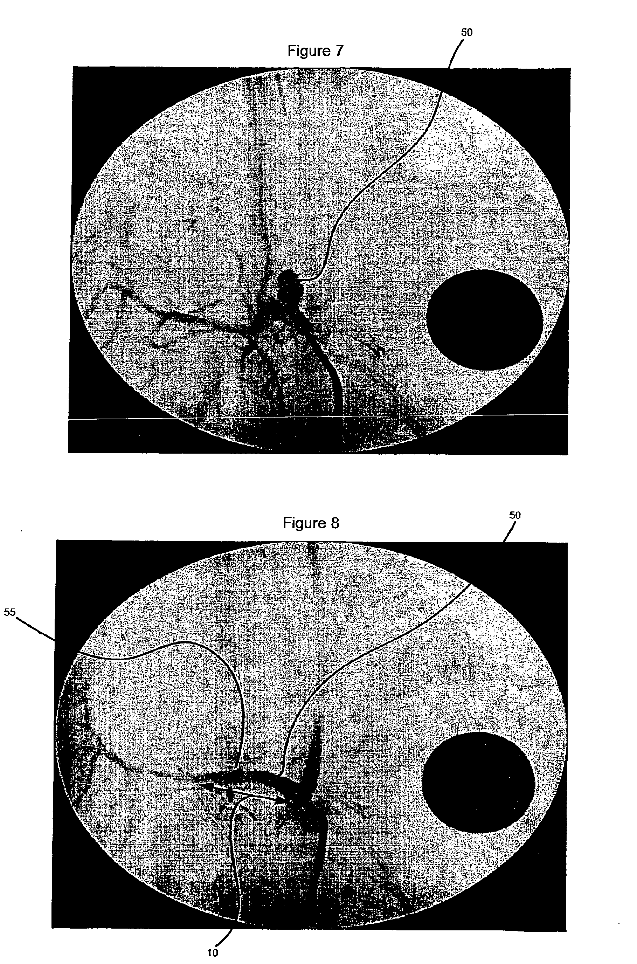

Figure 7, and the effect of the treatment;

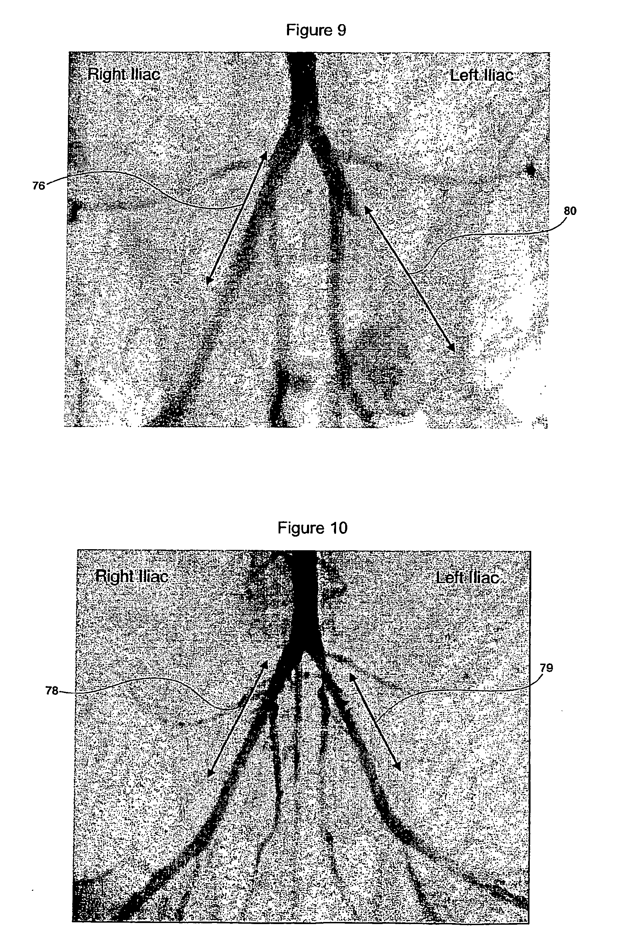

Figure 9 is an image of a chronic angiograph of iliac arteries showing the patency of vessels implanted with the endovascular device having a solid membrane made from a polyurethane based material with fluorocarbon surface-modified end groups;
and
Figure 10 is an image of a chronic angiograph of iliac arteries showing the patency of vessels implanted with the endovascular device having a porous membrane made from a polyurethane based material with fluorocarbon surface-modified end groups.

### Detailed Description of the Drawings

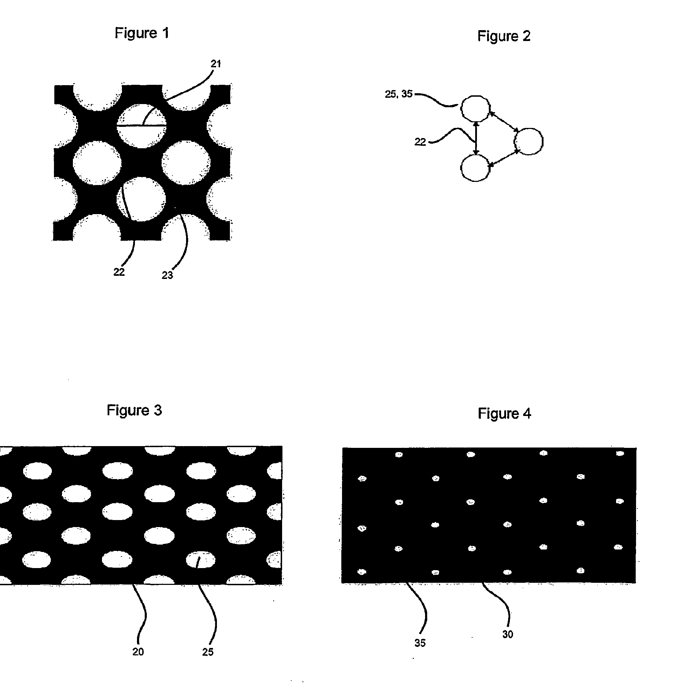

Referring to Figures 1 to 6, an endovascular device 10 for insertion into a bodily vessel 5 to treat a diseased, damaged or weakened portion 50 of a vessel wall is provided. The diseased, damaged or weakened portion 50 may be an aneurysm. The endovascular device 10 comprises a stent 11 and a membrane 20. The stent 11 may be balloon expandable or self-expandable. The membrane 20 covers at least a portion of the circumferential surface of the stent 11. The membrane 20 comprises a plurality of pores 25. The porosity of the membrane 20 is defined by the ratio of the material surface area of the membrane 20 which is determined by the size 21 of the pores 25 and the distance 22 between adjacent pores 25. The stent 11 is positioned in the vessel 5 such that the membrane 20 covers at least the diseased, damaged or weakened portion 50 of the vessel wall. The porosity of the membrane 20 obstructs blood supply to the diseased, damaged or weakened portion 50 of the vessel wall and enhances healing of the vessel 5.

The membrane 20 covers the stent 11 either partially or fully. The membrane 20 is porous and permeable containing specifically designed pore patterns. The membrane 20 is an ultra thin film and is made from a polyurethane-based polymer which has undergone special surface treatments and modifications to improve the biocompatibility of device 10, and enhance healing of the vessel 5 after the device 10 is implanted.

This device 10 may be used for the treatment of endovascular diseases such as aneurysms, AVMs and CC fistulas. The pore patterns are designed with consideration of factors such as specific flow conditions of blood vessels, location of the diseased vessel, for example, in the intracranial region, above and below ophthalmic artery, type of endovascular disease where the vessel wall is weakened or damaged, for example, saccular aneurysms, wide neck aneurysms, fusiform aneurysms, and CC fistula, and other peculiarities associated with the endovascular disease.

Several ways of improving the success of long term post implantation were implemented. The membrane 20 is a biomaterial called Biospan^{™}. The membrane 20 is fabricated as thin as possible to reduce as much as possible the bulk material placed in the vessel 5 to minimise any negative vessel reaction. However, the material of the membrane 20 must be made strong enough to obstruct blood circulation to the aneurysm 50 and withstand pulsatile blood pressure. The material is made porous and designed with a specific pattern of porosity. This approach helps to make the membrane 20 even less bulky in the vessel 5 and thus occupies less space. The specific pattern of porosity provides free communication between blood and vessel wall cells to enhance healing of the vessel 5, and also covers and isolates the aneurysm 50. The surface of the membrane 20 is molecularly altered with different end groups to isolate the material substrate of the membrane 20 from direct contact with blood, and cells' environment of the vessel wall. Different end groups were added to the material solution during its synthesis. The following end groups were used which covalently bonded to the substrate: Silicon, PEO (polyethylene oxide) and fluorocarbon.

The design of the membrane 20 is initially determined according to the application of the device 10. Next, three main factors are considered: pore size 21 and bridge dimension 22, 23, which contribute to the material ratio of the membrane 20.

Pore size 21 is measurable when the membrane 20 is in two different stages, namely, "as designed and manufactured" and "as deployed". A functional relationship exists where the "as manufactured" pore size is smaller than the "as deployed" pore size 21 by a factor of 1.5 to 2.5.

Bridge dimension 22, 23 refers to the shortest distance separating one pore 25 from its adjacent pores. Each pore 25 may be spaced from adjacent pores at varied distances. Preferably, equidistant porosity is desired as illustrated in Figure 2, which means all distances from one pore 25 to its adjacent pores are equal. Different bridge dimensions 22, 23 at different parts of the membrane 20 may be used, for example, circumferentially or longitudinally. Similar to pore size 21, bridge dimensions 22, 23 can also be measured in two functionally related stages: "as designed and manufactured" and "as deployed". The "as designed bridge dimension" is larger than the "as deployed" bridge dimension 22, 23 by a factor of 1 to 2.

Material ratio refers to the proportion of material coverage with respect to the total surface area of the membrane 20 with respect to the outer diameter of the stent 11. Material ratio may be represented in percentage form where the percentage material ratio is the same as the percentage material coverage, that is, 100% - %coverage = % porosity. Material ratio can also be expressed in "as manufactured" and "as deployed" stages. The functional relationship between the material ratio stages is a combination of pore size 21 and bridge dimension 22.

The main function of the membrane 20 is to cover the aneurysm neck and the damaged or weakened portion of vessel walls to repair and heal the diseased portion of the vessel wall. On the other hand, the membrane 20 allows all engaged vessels including adjacent micro-branches such as perforators 55 to stay patent after placement, if necessary.

Two examples of the device 10 are provided: macro-porous and micro-porous.

### Macro-porous

The macro-porous device 10 has a membrane 20 that is moderately to highly porous. One application for the macro-porous device 10 is to treat aneurysms within close proximity of branches or perforators 55. Another specific application is the treatment of an intracranial saccular or wide neck aneurysm 50 located above the ophthalmic artery where perforators 55 extend from the parent artery within close proximity of the aneurysm 50.

Aneurysms 50 are treated by the device 10 by reducing the volume, velocity and force of blood flowing into an aneurysm sac via the aneurysm neck. Having these reductions assist in the treatment of the aneurysm 50 in two ways. First, the risks of aneurysm sac enlargement or rupture are reduced. More importantly, disrupting blood flow in and out of the aneurysm sac and dispersing blood flow within the aneurysm 50 triggers intra-aneurysmal thrombosis. This leads to the obliteration of the aneurysm 50. The device 10 is able to induce aneurysm thrombosis but has sufficient porosity through the pores 25 of the membrane 20 to continue feeding vital perforators 55 and branch arteries.

Turning to Figure 3, the membrane 20 of the device 10 has pore sizes from about 20 to 150 µm and bridge dimensions from about 40 to 100µm (in either "as manufactured" or "as deployed" stages). The overall material ratio can range from about 25% to 75% for a macro-porous membrane 20. The thickness of the membrane 20 is from about 12,7µm to 50,8µm (0.0005" to 0.002")

Turning to Figures 7 and 8, the device 10 effectively reduces blood flow into an aneurysm 50. This encourages intra-aneurysmal thrombosis to occur. Figure 7 shows an aneurysm located in the subclavian artery of a rabbit. Figure 8 shows the result within a few hours after deployment of the device 10 in the vessel 5. Blood supply is substantially prevented from flowing into the aneurysm 50. At the same time, the pore pattern of the membrane 20 continues to allow a non-disruptive supply of blood through vital microscopic vessels (perforators) 55 located proximal to the deployed device 10.

The device 10 uses the antagonistic relationship between the sufficient reduction of blood supply to disrupt and thus heal an aneurysm 50 and the maintenance of sufficient blood supply vital to keep micro-branches (perforators) 55 patent.

For example, consider an aneurysm 50 with aneurysm neck diameter of 6mm and height of 10mm. If the aneurysm neck is covered by a 25% material ratio macro-porous device 10, a reduction of 25% blood flow into the aneurysm sac is possible. It is expected that the reduction in blood flow will exceed 25% due to the viscosity of blood as well as further reduction of blood flow due to flow disruption and dispersion. Up to 75% material ratio (% coverage) may be required to effectively stop blood circulation into an aneurysm 50 and for intra-aneurysmal thrombosis to occur. This however, is dependent on the geometry of the particular aneurysm 50.

Pore patterns for the membrane 20 are designed to partially cover but not occlude any perforators 55 that the macro-porous device 10 is positioned proximal to. In order to maintain the patency of branch/perforator arteries 55, less than 50% of the ostial diameter is covered. The diameter of the smallest perforators 55 is about 100 µm. Therefore, the bridge dimension 22, 23 for the membrane 20 is selected to be within 40 to 100 microns. During post expansion of the device 10, the pores 25 expand and the bridges 22, 23 narrow. Thus, there will be less than 50% coverage at even the smallest 100 µm perforators 55. The restriction to percentage material coverage is so that blood flow into the perforators 55 remains undisrupted and thrombotic reactions are not triggered.

Disruption of blood circulation into an aneurysm 50 and blood stagnation leads very quickly to blood coagulation and closing of the aneurysm 50. Consequently full vessel recovery may be achieved.

Generally, decreasing the flow rate of blood into and within the aneurysm 50 increases the chance of occluding the aneurysm 50. Covering the aneurysm 50 by more than 20% would affect the hemodynamics such that thrombosis is promoted to eventually occlude the aneurysm 50. This is dependent on the size and shape of the aneurysm 50. For example, larger and wider neck aneurysms 50 require increased coverage to affect the hemodynamics.

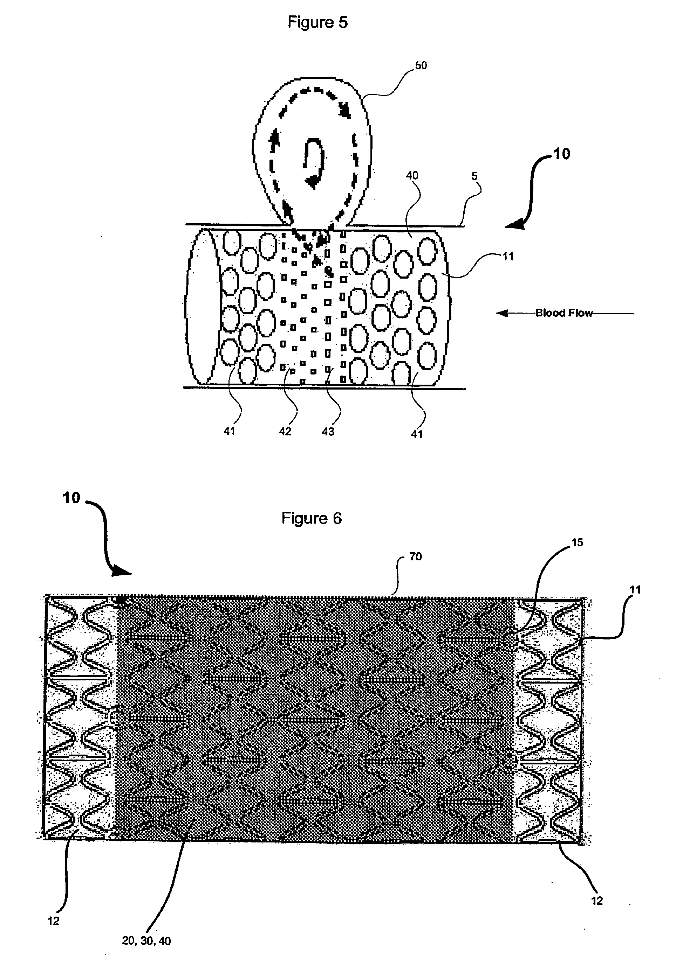

Turning to Figure 5, a slight reduction or disruption of blood supply into the aneurysm 50 due to a specific pore pattern of the membrane 20 positioned against the aneurysm neck disperses the high energy flow impact directed at the distal lateral wall of the aneurysm 50. It is at the distal lateral wall of the aneurysm 50 where most aneurysms 50 grow and rupture. Therefore, the membrane 20 will also lead to an elimination of potential rupture of the aneurysm 50.

The device 10 minimises potential trauma caused by implantation of the device 10 in the vessel 5. The porosity of the membrane 20 introduces less bulk and injury to the vessel 5. In addition, the specific pattern of porosity of the membrane 20 enhances healing by allowing cell migration and communication through the membrane 20.

In one example, the device 10 has a variable material ratio throughout the length and circumference of the membrane 20. This feature improves the performance and efficacy of the device 10 by healing the aneurysm 50 and keeps the parent vessel patent. Variations of the material ratio of the membrane 20 may include: higher material ratio at a distal part of the aneurysm neck, lower material ratio at a proximal part of the aneurysm neck, having the least material ratio at areas which are away from the aneurysm neck region, or a partially covered stent 11.

For a higher material ratio at a distal part of the aneurysm neck, even in pulsatile flow, blood flow into the aneurysm 50 through the aneurysm neck occurs mostly at distal part of the neck cross-sectional area. By increasing the material ratio by decreasing pore size 21 and increasing bridge dimensions 22, 23 of the membrane 40, less blood enters the aneurysm 50 and flow dynamics in the aneurysm 50 are disrupted. The distal part of aneurysm neck that is associated with aneurysm rupture also experiences less hemodynamic force as the impact of the blood flow is disrupted and dispersed by the membrane 40.

For a lower material ratio at a proximal part of the aneurysm neck, it may be beneficial to increase the porosity of the membrane 40 at the proximal part of the aneurysm neck, where outflow normally occurs. This facilitates better blood outflow from the aneurysm 50. The degree of material ratio is carefully controlled to prevent the disrupted blood flow from entering the aneurysm 50 at the proximal part instead. The change in material ratio from distal to proximal may also have to be incremental or progressive.

For the least material ratio at areas away from the aneurysm neck region, the material ratio is made as low as possible using large pore sizes 21 for the following two reasons: to allow blood flow into perforators 55 that may extend from the parent artery within close proximity of the aneurysm 50; and reducing the amount of material positioned away from the aneurysm neck and its immediate proximity improves biocompatibility. By reducing the material ratio in areas where high coverage is not needed, less net amount of polymer will be introduced into the body. Lesser material will result in lesser inflammatory and other immune responses. The large size of the pores 25 may also allow for better endothelial cell migration for faster endothelialization of the device 10.

For a partially covered stent 11, areas of the stent 11 that are not used as a scaffold for the membrane 20 may be left as a bare metallic scaffold to further reduce the material ratio. This further reduces chances of blockage of perforators 55 and improves biocompatibility of the device 10. For example, bare stent ends 12 ensure the proper placement of the device 10 in the vessel 5.

The device 10 facilitates healing of the aneurysm 50 while ensuring patency of parent arteries and perforators 55. Additional features involving the selection of suitable pore sizes 25 and bridge widths 22, 23 may be adopted to specific conditions. For example, aneurysms 50 of different types or geometry may require different pore patterns.

### Micro-porous

Referring to Figure 4, the micro-porous device 10 is used for conditions which require total coverage to immediately block blood flow, for example, CC fistula, or where there is little or no risk of blocking perforators 55, for example, below the ophthalmic artery.

The micro-porous device 10 functions similar to a macro-porous device 10, except that the micro-porous device 10 uses a membrane 30 that is significantly less porous as illustrated in Figure 4. The device 10 is used in areas where blockage of small vessels near the diseased vessel wall is not an issue. Patient survivorship totally depends on effective blockage of the aneurysm 50 and porosity may enhance healing process and also endothelialization.

The membrane 30 of the micro-porous device 10 has pore sizes from about 1 to 40 µm and bridge dimensions from about 10 to 100µm in either "as manufactured" or "as deployed" stages. The overall material ratio ranges from about 75% to 100% for a micro-porous membrane 30.

The micro-porous device 10 has a high degree of coverage which significantly reduces or completely stops blood flow through the membrane 30. The pores 35 are small enough to affect the surface tension of blood flow in such a way that blood is prevented from flowing through the pores 35 in contrast to the pores 25 of the membrane 20 of the macro-porous device 10. Even though the size of the pores 35 is very small, they enable better endothelial cell migration and tissue in-growth through for faster endothelialization and healing of the vessel 5.

Similarly, the micro-porous device 10 may also be partially covered by having uncovered ends 12 of the stent 11 to reduce material use and enhance biocompatibility.

### Lubricious coating for membrane and/or stent

A lubricious layer 70 can be applied onto the outer surface of the stent 11 for improved trackability during delivery of the device 10 to the surgical site. This coating 70 may be applied after the device 10 is fabricated and placed onto a delivery system or before placement onto the delivery system. Alternatively, this layer 70 may be introduced in combination with the membrane material as an additional surface property. That is, to modify the chemical structure or surface characteristics of the membrane 20 to achieve an inherently low coefficient of friction on surface. The layer 70 is biocompatible and has properties that will reduce the coefficient of friction between the stent 11 and/or membrane 20 and vessel walls during tracking. The layer 70 also has the right balance of stability and durability to maintain integrity during tracking. This layer 70 may be applied to both macro and micro-porous devices 10.

The lubricious layer 70 may be made from, for example, hydrophilic PVP (polyvinylpyrrolidone) to reduce friction during tracking. Other hydrophilic polymers like polyacrylate or polymethacrylate as well as hydrogels like PEO (polyethylene oxide) may also be used. Gelatin may also be used. These polymers may also be used to modify the membrane material to achieve predetermined surface properties of the membrane 20.

### Stent markers

Turning to Figure 6, special stent markers 15 may be incorporated into the stent 11. This enables accurate alignment of the high material ratio (high %coverage) portion of the membrane 20 against the aneurysm neck. Precise alignment is important for effective treatment of aneurysms 50 in areas known to have micro-branches, side-branches or perforators 55.

### Material of the membrane

The membrane 20 is made from biocompatible, highly elastomeric polymer. Polyether urethane (PEU) or polycarbonate urethane (PCU) may be used. Trade names for PEU include: Tecoflex Tecothane^{™}, Hapflex^{™}, Cardiothane^{™}, Pellethane^{™}, and Biospan^{™}. Trade names for PCU include: ChronoFlex^{™}, Carbothane^{™}, and Corethane^{™}.

In another example, the membrane 20 may be non-porous. The membrane 20 is made from BioSpan F^{™}, a material developed by Polymer Technology Group (PTG), Berkeley, California, USA. BioSpan F^{™} is a polyurethane based material with fluorocarbon surface-modified end groups. During in-vitro and in vivo studies, this material possesses excellent compatibility properties matching the environment of small blood vessels. The selection of BioSpan F^{™} for the membrane 20 of the device 10 in treating small vessels is preferred due to its anti-thrombogenic and healing properties. Preferably, the membrane 20 has a specific pore pattern as described earlier to obtain better results. This is confirmed by a series of in-vitro and in-vivo experiments, comparing different materials for the membrane 20.

BioSpan^{™} is a suitable material for the membrane 20 and may have some additional surface alteration or treatment to enhance acceptance of the material in the vessel environment. Several surface and material modifications were used for in-vivo and in-vitro experiments.

Referring to table below, initial in-vitro biocompatibility tests have shown that when comparing three materials: BioSpan^{™} and ePTFE, BioSpan F^{™} was the least thrombogenic as illustrated below. Turning to Figure 9, further animal studies confirm superior biocompatibility using BioSpan F^{™}. An endovascular device 76 with a membrane made from BioSpan F^{™} is shown on the right iliac arteries (left side of the figure) which shows the vessel 5 remains patent with minimal narrowing. BioSpan F^{™} also exhibited an unexpectedly good healing response compared to all other types of material and variations of BioSpan^{™}. For comparison, an endovascular device 80 with a membrane made from a control material is shown on left iliac arteries (right side of the figure) which shows total occlusion of the vessel 5.

### Summary of protein adsorption test (Namsa, 7 September 2005)

| Test article | Concentration of protein found (µg/ml) | Amount of protein (µg) | Adsorbed protein (µg/cm²) | Adsorbed protein (µg/g) |
|---|---|---|---|---|
| BioSpan | 5.5 | 28 | 1.4 | 230 |
| BioSpan F | 3.5 | 18 | 0.88 | 160 |
| ePTFE | 16 | 80 | 4.0 | 4600 |

Referring to Figure 10, the animal studies also showed that when BioSpan F^{™} was used, the membrane 20 with specifically designed and manufactured porosity pattern had a lower degree of narrowing and thus had better healing properties than the solid covered stent. The animal study showed that BioSpan F^{™} was a material suitable to be used for a small vessel endovascular device 10. An endovascular device 78 with a porous membrane made from BioSpan F^{™} is shown on the right iliac arteries (left side of the figure) which shows less than 5% narrowing of the vessel 5. An endovascular device 79 with a non-porous/solid membrane made from BioSpan F^{™} is shown on the left iliac arteries (right side of the figure) which shows 15 to 20% narrowing of the vessel 5.

Other materials include variations of the BioSpan^{™} family using the same surface modifying end group technique, but with application of different end groups. These variations include BioSpan PS^{™}. BioSpan PS^{™} is surface modified material with PEO and silicon end groups.

It will be appreciated by persons skilled in the art that numerous variations and/or modifications may be made to the invention within the scope of the claims The present embodiments are, therefore, to be considered in all respects illustrative and not restrictive.

## Claims

1. An endovascular device (10) for insertion into a bodily vessel to treat a diseased, damaged or weakened portion of a vessel wall, the endovascular device comprising:
a mechanically expandable device (11) expandable from a first position to a second position, said mechanically expandable device is expanded radially outwardly to the second position such that the circumferential surface of said mechanically expandable device engages with the inner surface of the vessel so as to maintain a fluid pathway through said vessel; and
a membrane (20) covering at least a portion of the circumferential surface of said mechanically expandable device;
wherein the membrane has equidistant porosity and pore (25) sizes from about 1 to 40 µm, bridge dimensions (22) from about 10 to 100 µm, and a material ratio, that is a proportion of material coverage with respect to total surface area of the membrane, from 75% to 100% ; and
wherein the membrane is made from a biocompatible highly elastomeric polymer, the polymer being a polyurethane based material with end groups.

2. The device according to claim 1, wherein the polyurethane based material has undergone a surface treatment such that the end group is any one from the group consisting of: fluorocarbon surface-modified end groups, and polyethylenglycol and silicon surface-modified end groups.

3. The device according to claim 1, further comprising a lubricious layer applied to the exterior surface of the membrane and/or exterior circumferential surface of the mechanically expandable device to reduce friction between the membrane and/or mechanically expandable device with the vessel wall of the bodily vessel.

4. The device according to claim 3 wherein the lubricious layer is made from a polymer from any one from the group consisting of: hydrophilic polyvinylpyrrolidone, polyacrylate, polymethacrylate, hydrogels, polyethylene oxide and gelatin.

5. The device according to claim 1, further comprising radiopaque markers positioned on the mechanically expandable device to enable alignment of the membrane to the diseased, damaged or weakened portion of the vessel wall.

6. The device according to claim 1, wherein the device is configured to be inserted into a diseased, damaged or weakened portion of the vessel wall that is any one from the group consisting of: intracranial aneurysm, saccular aneurysm, wide neck aneurysm, fusiform aneurysm, caroticocavernous fistula, and arteriovenous malformation (AVM).

7. The device according to claim 1, wherein the device is configured to be inserted into a bodily vessel that has an inner diameter of about 2.0 to 4.5mm.

## Patentansprüche

1. Ein endovaskuläres Instrument (10) zur Einführung in ein Körpergefäß zur Behandlung eines erkrankten, beschädigten oder geschwächten Teils einer Gefäßwand bestehend aus:
einer mechanisch erweiterbares Vorrichtung (11), die von einer ersten zu einer zweien Position erweitert werden kann, wobei die besagte mechanisch erweiterbare Vorrichtung radial nach außen in die zweite Position erweitert wird, so dass die Umfangsfläche der besagten mechanisch erweiterbares Vorrichtung solchermaßen die Innenfläche des Gefäßes erweitert, dass ein Weg für Flüssigkeiten durch das besagte Gefäß aufrechterhalten wird: und
eine Membran (20), die mindestens einen Teil der Umfangsfläche der besagten mechanisch erweiterbaren Vorrichtung abdeckt;
**dadurch gekennzeichnet, dass** die Membran äquidistante Porosität und Poren (25) in Größen von etwa 1 bis 40 µm, Porenabstände (22) von etwa 10 bis 100 µm und eine Materialdeckung aufweist, die 75 % bis 100 % der Gesamtoberfläche der Membran beträgt; und
**dadurch gekennzeichnet, dass** die Membran aus einem biokompatiblen, elastomerischen Polymer besteht, wobei es sich bei diesem Polymer um ein auf Polyurethan basiertem Material mit Endgruppen handelt.

2. Das Instrument nach Anspruch 1, **dadurch gekennzeichnet, dass** das auf Polyurethan basierte Material einer Oberflächenbehandlung unterzogen wurde, so dass die Endgruppe aus einer aus der Gruppe bestehend aus oberflächenmodifizierten Endgruppen auf Basis von Fluorkohlenstoff und oberflächenmodifizierten Endgruppen auf Basis von Polyethylenglycol und Silicon ausgewählte Endgruppe besteht.

3. Das Instrument nach Anspruch 1, weiter bestehend aus einer schmierigen, auf der Außenfläche der Membran bzw. der außenliegenden Umfangsfläche der besagten mechanisch erweiterbares Vorrichtung aufgetragenen Schicht zur Reduzierung der Reibung zwischen der Membran bzw. der mechanisch erweiterbares Vorrichtung und der Gefäßwand des Körpergefäßes.

4. Das Instrument nach Anspruch 3, **dadurch gekennzeichnet, dass** die schmierige Schicht aus einem aus der Gruppe bestehend aus hydrophilem Polyvinylpyrrolidon, Polyacrylat, Polymethacrylat, Hydrogel, Polyethylenoxid und Gelatine ausgewählte Polymer besteht.

5. Das Instrument nach Anspruch 1, weiter bestehend aus radiopaken, auf der mechanisch erweiterbaren Vorrichtung positionierten Markern, die die Anpassung der Membran an den erkrankten, beschädigten oder geschwächte Teil der Gefäßwand ermöglichen.

6. Das Instrument nach Anspruch 1, **dadurch gekennzeichnet, dass** das Instrument so konfiguriert ist, dass es in einen erkrankten, beschädigten oder geschwächten Teil einer Gefäßwand, ausgewählt aus der Gruppe bestehend aus intrakraniellen Aneurysmen, sackförmigen Aneurysmen, Aneurysmen mit weitem Hals, fusiformen Aneurysmen, Carotis-Cavernosus-Fisteln und arterio-venösen Malformationen (AVM), eingeführt werden kann.

7. Das Instrument nach Anspruch 1, **dadurch gekennzeichnet, dass** das Instrument in ein Körpergefäß mit Innendurchmesser von etwa 2,0 mm bis 4,5 mm eingeführt werden kann.

## Revendications

1. Un dispositif endovasculaire (10) pouvant être inséré dans un vaisseau pour traiter une partie malade, endommagée ou affaiblie de la paroi d'un vaisseau, ce dispositif endovasculaire comprenant :
Un dispositif à expansion mécanique (11) permettant une expansion d'une première position à une deuxième position, ledit dispositif à expansion mécanique se dilatant radialement vers l'extérieur jusqu'à la deuxième position de sorte que la surface circonférentielle dudit dispositif à expansion mécanique s'engage avec la surface interne du vaisseau de façon à maintenir un chemin de fluide dans ledit vaisseau ; et
une membrane (20) couvrant au minimum une partie de la surface circonférentielle dudit dispositif à expansion mécanique ;
dans lequel la membrane présente une porosité équidistante et différentes grosseurs de pores (25) comprises entre 1 et 40 µm, des dimensions de pont (22) comprise entre 10 et 100 µm, et un ratio de matériau proportionnel à la couverture de matériau relativement à la superficie totale de la membrane, de 75% à 100%.
dans laquelle la membrane est produite avec un polymère biocompatible extrêmement élastomère, le polymère étant un matériau à base de polyuréthane avec des groupes terminaux.

2. Le dispositif selon la revendication 1, dans lequel le matériau à base de polyuréthane a été soumis à un traitement superficiel de sorte que le groupe terminal appartient à un quelconque des groupes suivants : groupes terminaux à surface modifiée au fluorocarbure ; et groupes terminaux à surface modifiée au polyéthylèneglycole et silicone.

3. Le dispositif selon la revendication 1, comprenant également une couche lubrifiante appliquée sur la surface extérieure de la membrane et/ou la surface circonférentielle extérieure du dispositif à expansion mécanique avec la paroi du vaisseau pour réduire la friction entre la membrane et/ou le dispositif à expansion mécanique et la paroi du vaisseau.

4. Le dispositif selon la revendication 3, dans lequel la couche lubrifiante est composée d'un polymère appartenant à un quelconque des éléments du groupe composé de : polyvinylepyrrolidone hydrophile, polyacrylate, polyméthylacrylate, hydrogels, oxyde de polyéthylène, et gélatine.

5. Le dispositif selon la revendication 1, comprenant également des repères radiopaques placés sur le dispositif à expansion mécanique afin de permettre l'alignement de la membrane avec la partie malade, endommagée ou affaiblie du de la paroi du vaisseau.

6. Le dispositif selon la revendication 1, dans lequel le dispositif est configuré pour être inséré dans une partie malade, endommagée ou affaiblie du de la paroi du vaisseau appartenant à un quelconque des composants du groupe suivant :
anévrisme intracrânien, anévrisme sacciforme, anévrisme à col large, anévrisme fusiforme, fistule carotido-caverneuse, et déformation artério-veineuse (AVM).

7. Le dispositif selon la revendication 1, dans lequel le dispositif est configuré pour être inséré dans un vaisseau au diamètre compris entre 2,0 et 4,5 mm environ.
